(19) Europäisches Patentamt
European Patent Office
Office européen des brevets

(11) **EP 4 134 963 A1**

(12) **EUROPEAN PATENT APPLICATION**

(43) Date of publication:
**15.02.2023   Bulletin 2023/07**

(21) Application number: **22186188.3**

(22) Date of filing: **21.07.2022**

(51) International Patent Classification (IPC):
**G16C 20/30** (2019.01)    **G16C 20/70** (2019.01)
**G06N 20/10** (2019.01)

(52) Cooperative Patent Classification (CPC):
**G16C 20/30;** G06N 20/10; G16C 20/70

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:   **09.08.2021   CN 202110909058**

(71) Applicant: **Shanghai Totole Food Ltd.
Shanghai Shanghai (CN)**

(72) Inventors:
  • **ZHANG, Jiahui
    Shanghai (CN)**
  • **WANG, Fang
    Shanghai (CN)**
  • **LI, Xiaoyan
    Shanghai (CN)**
  • **LIU, Taiang
    Shanghai (CN)**
  • **WANG, Xichang
    Shanghai (CN)**
  • **GE, Xiaotong
    Shanghai (CN)**
  • **YU, Zheng
    Shanghai (CN)**

(74) Representative: **Patentanwälte Gierlich &
Pischitzis
Partnerschaft mbB
Gerbermühlstraße 11
60594 Frankfurt am Main (DE)**

(54) **QUANTITATIVE PREDICTION METHOD FOR SENSORY EVALUATION SCORE OF DELICIOUS DEGREE AND LIQUID GENERATING SENSE OF SOLID-STATE COMPOUND SEASONING**

(57)    The present invention provides a quantitative prediction method for a sensory evaluation score of a delicious degree and a liquid generating sense of a solid-state compound seasoning. The contents of 7 components IMP, GMP, $Na^+$, Asp, Glu, Gly and Ala in a plurality of solid-state compound seasonings are used as independent variables; each solid-state compound seasoning is artificially scored for the delicious degree and the liquid generating sense, and a formula is used to comprehensively process the contents of 7 independent variables; comprehensively processed data and the liquid generating sense are quantitatively modeled, to generate a quantitative prediction model for sensory evaluation of the delicious degree and the liquid generating sense of the solid-state compound seasoning; and after the contents of 7 independent variables in the solid-state compound seasoning to be detected are comprehensively processed, it is substituted into the quantitative prediction model, to obtain the sensory evaluation score of the delicious degree and the liquid generating sense of the solid-state compound seasoning to be detected. Compared with artificial evaluation, the method of the present invention has a more objective, accurate and convenient predicted result.

## Description

## Technical Field

The present invention relates to the technical field of sensory evaluation of seasonings, in particular to a quantitative prediction method for a sensory evaluation score of a delicious degree and a liquid generating sense of a solid-state compound seasoning.

## Background

A solid-state compound seasoning is compounded by two or more seasonings, and a main function of the compound seasoning is to season and impart a special flavor to food and dishes. In recent years, the seasoning industry in China makes a great progress, the requirements of people for the seasonings are gradually shifted from a single demand to diversification, and solid-state compound seasoning products become more and more popular in the market. However, the sensory evaluation of the solid-state compound seasonings is currently mainly conducted by tasters who taste these seasonings, and then give sensory evaluation scores based on their own feelings and accumulated experience in the past. The sensory evaluation scores given in this way often have strong personal subjectivity. The different tasters taste the same solid-state compound seasoning, the sensory evaluation scores given are often different, and some have relatively large differences. For example, some tasters give 1.5 scores while some tasters give 3.0 scores for a delicious degree and a liquid generating sense of a certain solid-state compound seasoning. There are many reasons for this, it may be that the different tasters have the accumulated experience in different times, some tasters have the accumulated experience for more than ten years, and some tasters have the accumulated experience for only three or four years. It may also be related to ages, preferences, physical and mental states of the tasters, some tasters may be sensitive to the liquid generating sense, and some tasters may have problems for their bodies while they taste, and their spirit and taste sensations may be dull and the like. These reasons all lead to the differences in the sensory evaluation of the solid-state compound seasonings. However, due to the more and more extensive applications of the compound seasonings in daily life of people, the requirements of people for delicious taste are also higher and higher, and the requirements for the accuracy of their sensory evaluation are higher and higher. How to find a more objective sensory evaluation scoring method to gradually replace the artificial subjective scoring method becomes an urgent task for those skilled in the art.

## Summary of the Invention

A purpose of the present invention is to propose a quantitative prediction method for giving an objective and accurate score to a delicious degree and a liquid generating sense of a compound seasoning.

In order to achieve the above purpose, the present invention provides a quantitative prediction method for a sensory evaluation score of a delicious degree and a liquid generating sense of a solid-state compound seasoning. The contents of 7 components IMP, GMP, $Na^+$, Asp, Glu, Gly and Ala in a plurality of solid-state compound seasonings are used as independent variables; each solid-state compound seasoning is artificially scored for the delicious degree and the liquid generating sense, and a formula is used to comprehensively process the contents of 7 independent variables; comprehensively processed data and the delicious degree and liquid generating sense are quantitatively modeled, to generate a quantitative prediction model for sensory evaluation of the delicious degree and the liquid generating sense of the solid-state compound seasoning; and

after the contents of 7 independent variables in the solid-state compound seasoning to be detected are comprehensively processed, it is substituted into the quantitative prediction model, to obtain the sensory evaluation score of the delicious degree and the liquid generating sense of the solid-state compound seasoning to be detected.

Further, the comprehensive processing includes using the following conversion equation to process the parameter content:

$$X_1 = +0.260[IMP] + 4.955E\text{-}2[GMP] + 1.971E\text{-}2[Na] + 0.271[Asp] - 2.521E\text{-}3[Glu] + 0.453[Gly] + 1.169[Ala] - 0.515;$$

$$X_2=+0.795[IMP]+0.340[GMP]+0.133[Na]-0.617[Asp]-4.217E\text{-}3[Glu]-0.765[Gly]+0.806[Ala]-1.488;$$

$$X_3=+2.354[IMP]+1.660[GMP]-0.103[Na]-0.340[Asp]+2.758E\text{-}3[Glu]-0.663[Gly]+0.686[Ala]-0.862;$$

$$X_4=-0.257[IMP]-1.850[GMP]-0.179[Na]-3.978E\text{-}2[Asp]+3.134E\text{-}3[Glu]-1.035[Gly]+0.665[Ala]+3.013;$$

$$X_5=+2.457[IMP]-2.734[GMP]+0.130[Na]+0.169[Asp]+2.082E\text{-}3[Glu]-0.106[Gly]+6.545E\text{-}2[Ala]-3.024;$$

$$X_6=+5.119[IMP]-5.251[GMP]-6.594E\text{-}2[Na]-5.659E\text{-}2[Asp]-9.137E\text{-}4[Glu]+0.132[Gly]-0.122[Ala]+1.209;$$

$$X_7=+0.917[IMP]+0.463[GMP]-2.781E\text{-}2[Na]+1.008[Asp]-3.006E\text{-}3[Glu]-0.719[Gly]-0.216[Ala]+0.754;$$

in the formula, $X_1$, $X_2$, $X_3$, $X_4$, $X_5$, $X_6$ and $X_7$ respectively represent 7 modeling data after the comprehensive processing of each solid-state compound seasoning.

Further, before the modeling, the number of the solid-state compound seasonings is greater than or equal to 70, and a taster tastes each solid-state compound seasoning and scores for the delicious degree and the liquid generating sense.

Further, in each solid-state compound seasoning, 7 independent variable data after the comprehensive processing and the delicious degree and the liquid generating sense are quantitatively modeled using a support vector machine regression algorithm.

Further, in the support vector machine regression algorithm, a radial basis kernel function is selected, a penalty factor is selected as 13, and an insensitive function is selected as 0.06.

Compared with the prior art, the advantages of the present invention are as follows:

1. The present invention may achieve the prediction of the sensory evaluation score of the delicious degree and the liquid generating sense of the solid-state compound seasoning, and provide data support for enterprises to monitor product quality.

2. In the process of predicting the sensory evaluation score of the delicious degree and the liquid generating sense of a new solid-state compound seasoning, the participation of tasters is no longer required, the subjective factors of the tasters are reduced, and predicted results are more objective and accurate.

3. Compared with artificial scoring, the whole scoring process of the present invention reduces the waste of samples, and improves the economic benefits of the enterprises.

4. The value of the delicious degree and the liquid generating sense measured by the modeling of the present invention is almost the same as the actual value, and the accuracy is higher.

**Brief Description of the Drawings**

FIG. 1 is a modeling result diagram of a support vector machine regression model of a sensory evaluation score of a liquid generating sense of a solid-state compound seasoning.
FIG. 2 is a leave-one-out cross-validation result diagram of a support vector machine regression model of a sensory evaluation score of a liquid generating sense of a solid-state compound seasoning.

**Detailed Description of the Preferred Embodiments**

In order to make purposes, technical schemes and advantages of the present invention clearer, the technical schemes of the present invention are further described below.
The present invention provides a quantitative prediction method for a sensory evaluation score of a delicious degree and a liquid generating sense of a solid-state compound seasoning, and the specific method is as follows:
Step 1: 70 solid-state compound seasonings are collected from the market, and the contents of IMP, GMP, $Na^+$, Asp, Glu, Gly and Ala in these 70 solid-state compound seasonings are detected in a laboratory, as independent variables. A taster then artificially scores the delicious degree and the liquid generating sense of these 70 solid-state compound seasonings, thus a 7*70 data matrix is constructed. In this data matrix, the scores of the delicious degree and the liquid generating sense are dependent variables, and the values of the contents of 7 components are the independent variables. In this way, basic data is obtained. A part of the data is shown in Table 1:

Table 1: Example data for component content and delicious degree and liquid generating sense

| Delicious degree Liquid generating sense | IMP | GMP | $Na^+$ | Asp | Glu | Gly | Ala |
|---|---|---|---|---|---|---|---|
| 2.9643 | 0.5672 | 0.4970 | 20.7440 | 0.1080 | 327.6768 | 0.0611 | 0.3570 |
| 2.3462 | 0.9210 | 0.9044 | 19.8099 | 0.0644 | 456.1787 | 0.0000 | 0.1143 |
| 2.9667 | 0.4029 | 0.3562 | 18.1632 | 0.2622 | 339.5147 | 0.1434 | 0.4971 |
| 2.6000 | 0.2083 | 0.1925 | 17.5965 | 0.0000 | 324.5237 | 0.0000 | 0.1292 |
| 3.2500 | 0.7506 | 0.6505 | 15.6197 | 1.1856 | 3.7781 | 0.3744 | 1.8953 |
| 2.8333 | 0.5166 | 0.5275 | 14.9193 | 0.0000 | 395.7990 | 0.1369 | 0.3618 |
| 3.1000 | 0.6628 | 0.5719 | 18.1564 | 0.0399 | 125.8107 | 0.0438 | 0.0000 |
| 2.7692 | 0.3007 | 0.2843 | 13.9500 | 0.1886 | 154.6255 | 0.0879 | 0.2371 |
| 2.8333 | 0.1769 | 0.0433 | 13.9232 | 0.4885 | 146.9194 | 0.5061 | 1.5095 |
| 2.5357 | 0.4184 | 0.4217 | 15.3087 | 0.0982 | 472.2619 | 0.0318 | 0.1546 |
| 2.9615 | 0.2630 | 0.2311 | 16.5982 | 0.2579 | 168.9501 | 0.1195 | 0.6884 |
| 2.5714 | 0.2147 | 0.1957 | 15.1796 | 0.0000 | 235.2053 | 0.0804 | 0.0998 |
| 2.4231 | 1.2045 | 1.1478 | 16.4784 | 0.0413 | 305.8874 | 0.1274 | 0.1953 |
| 2.7000 | 0.5078 | 0.4363 | 19.0262 | 0.0000 | 322.2571 | 0.0298 | 0.1951 |

Step 2: the independent variables in the basic data are comprehensively processed, and new variables after the comprehensive processing contain all the original independent variables. A conversion equation is as follows:

$$X_1=+0.260[IMP]+4.955E\text{-}2[GMP]+1.971E\text{-}2[Na]+0.271[Asp]-2.521E\text{-}3[Glu]+0$$

$$.453[Gly]+1.169[Ala]-0.515$$

$X_2 = +0.795[IMP] + 0.340[GMP] + 0.133[Na] - 0.617[Asp] - 4.217E-3[Glu] - 0.765[Gly] + 0.806[Ala] - 1.488$

$X_3 = +2.354[IMP] + 1.660[GMP] - 0.103[Na] - 0.340[Asp] + 2.758E-3[Glu] - 0.663[Gly] + 0.686[Ala] - 0.862$

$X_4 = -0.257[IMP] - 1.850[GMP] - 0.179[Na] - 3.978E-2[Asp] + 3.134E-3[Glu] - 1.035[Gly] + 0.665[Ala] + 3.013$

$X_5 = +2.457[IMP] - 2.734[GMP] + 0.130[Na] + 0.169[Asp] + 2.082E-3[Glu] - 0.106[Gly] + 6.545E-2[Ala] - 3.024$

$X_6 = +5.119[IMP] - 5.251[GMP] - 6.594E-2[Na] - 5.659E-2[Asp] - 9.137E-4[Glu] + 0.132[Gly] - 0.122[Ala] + 1.209$

$X_7 = +0.917[IMP] + 0.463[GMP] - 2.781E-2[Na] + 1.008[Asp] - 3.006E-3[Glu] - 0.719[Gly] - 0.216[Ala] + 0.754$

Example data after the comprehensive processing is shown in Table 2.

Table 2: Example data after comprehensive processing

| Delicious degree Liquid generating sense | $X_1$ | $X_2$ | $X_3$ | $X_4$ | $X_5$ | $X_6$ | $X_7$ |
|---|---|---|---|---|---|---|---|
| 2.9643 | -0.2857 | 0.6895 | 0.2235 | -0.5654 | 0.4329 | -0.2066 | -0.0698 |
| 2.3462 | -0.8389 | 0.3207 | 2.0723 | -0.9368 | 0.3173 | -0.5664 | 0.1355 |
| 2.9667 | -0.1735 | 0.0720 | -0.1082 | 0.2379 | 0.1289 | -0.1638 | -0.1835 |
| 2.6000 | -0.7716 | -0.1757 | -0.8890 | 0.5593 | -0.0600 | -0.2086 | -0.4585 |
| 3.2500 | 2.7165 | 1.9059 | 1.0288 | -0.3394 | -0.6291 | 0.3527 | 1.8137 |
| 2.8333 | -0.5733 | -0.3916 | 0.9351 | 0.5754 | -0.4184 | -0.2882 | -0.3093 |
| 3.1000 | -0.2429 | 1.0649 | 0.0735 | -1.1152 | -0.3278 | 0.2897 | 0.7522 |
| 2.7692 | -0.1697 | 0.0628 | -0.6587 | 0.4587 | -0.8836 | 0.1659 | 0.3843 |
| 2.8333 | 1.5628 | 0.4319 | -0.8747 | 1.3186 | -0.4587 | 0.6894 | -0.0905 |
| 2.5357 | -1.0523 | -0.9233 | 0.5930 | 0.9335 | -0.1462 | -0.3250 | -0.4696 |
| 2.9615 | 0.3946 | 0.6041 | -0.8050 | 0.4027 | -0.4178 | 0.0096 | 0.1580 |
| 2.5714 | -0.5902 | -0.2002 | -0.9384 | 0.6014 | -0.5646 | 0.0626 | -0.1670 |

(continued)

| Delicious degree Liquid generating sense | $X_1$ | $X_2$ | $X_3$ | $X_4$ | $X_5$ | $X_6$ | $X_7$ |
|---|---|---|---|---|---|---|---|
| 2.4231 | -0.2938 | 0.8006 | 3.0529 | -1.4123 | -0.4111 | -0.0279 | 0.9201 |
| 2.7000 | -0.5571 | 0.3756 | 0.0920 | -0.2186 | 0.1921 | -0.0519 | -0.1396 |

Step 3: support vector machine regression is used to quantitatively model the data after the comprehensive processing, to obtain a quantitative prediction model for a sensory evaluation score of the delicious degree and the liquid generating sense of the solid-state compound seasoning.

Step 4: 4 new solid-state compound seasonings are collected, and the contents of IMP, GMP, $Na^+$, Asp, Glu, Gly and Ala thereof are detected in the laboratory, but the taster no longer tastes artificially to give the score of the delicious degree and the liquid generating sense, and an independent test set is formed in this way. New sample data is collected as shown in Table 3:

Table 3: New data for independent test set

| Sample number | IMP | GMP | Na | Asp | Glu | Gly | Ala |
|---|---|---|---|---|---|---|---|
| 1 | 0.1804 | 0.0946 | 17.3588 | 0.3692 | 118.3176 | 0.1758 | 1.1325 |
| 2 | 0.3909 | 0.4091 | 22.8143 | 0.0578 | 340.8327 | 0.0116 | 0.2526 |
| 3 | 1.1456 | 1.0591 | 14.4801 | 0.0000 | 511.9885 | 0.0669 | 0.3426 |
| 4 | 0.4227 | 0.3909 | 18.9226 | 2.5703 | 346.2099 | 0.8600 | 1.4982 |

Step 5: the collected new data is substituted into the conversion equation, to obtain data after comprehensive mapping processing. Converted data is shown in Table 4.

Table 4: Some examples after comprehensive processing of new data

| $X_1$ | $X_2$ | $X_3$ | $X_4$ | $X_5$ | $X_6$ | $X_7$ |
|---|---|---|---|---|---|---|
| 1.0837 | 1.0532 | -1.2147 | 0.6144 | -0.2118 | 0.2467 | 0.1258 |
| -0.4864 | 0.7246 | -0.5372 | -0.7026 | 0.5274 | -0.7870 | -0.3617 |
| -0.7389 | -0.2215 | 3.6973 | -0.0676 | -0.1357 | 0.0560 | 0.2312 |
| 1.9510 | -0.9925 | -0.6365 | -0.1138 | 0.5756 | -0.4588 | 1.4037 |

Step 6: then, the data after the comprehensive processing is directly substituted into the quantitative prediction model for the sensory evaluation score of the delicious degree and the liquid generating sense of the solid-state compound seasoning, and it is predicted to obtain a sensory evaluation score of the delicious degree and the liquid generating sense of the solid-state compound seasoning in the independent test set.

The sensory evaluation score of the delicious degree and the liquid generating sense of the solid-state compound seasoning in the independent test set is independently tested by a support vector machine regression model, and its results are shown in Table 5.

Table 5: Prediction result

| Sample number | Score of delicious degree and liquid generating sense |
|---|---|
| 1 | 3.0373 |
| 2 | 2.8018 |
| 3 | 2.4994 |
| 4 | 2.7460 |

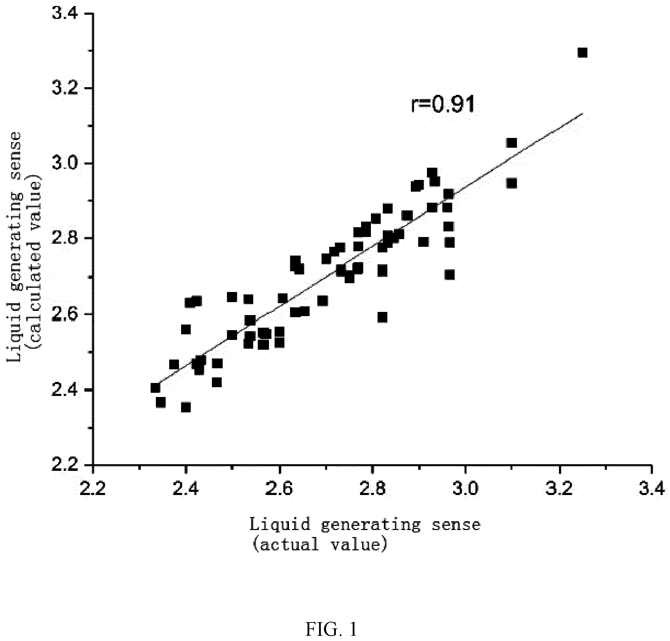

For 70 data, modeling results of the support vector machine regression model for the sensory evaluation score of the delicious degree and the liquid generating sense of the solid-state compound seasoning are shown in FIG. 1.

It may be seen from FIG. 1 that a correlation coefficient between the actual value and the calculated value of the liquid generating sense is 0.91, the value is very high, the accuracy of the model is relatively high, and the established model is high in reliability.

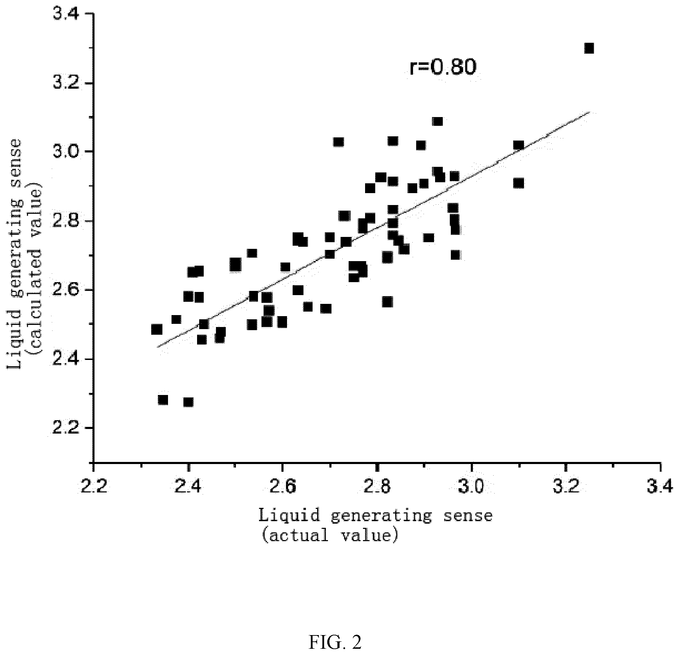

For 70 data, leave-one-out cross-validation results of the support vector machine regression model for the sensory evaluation score of the delicious degree and the liquid generating sense of the solid-state compound seasoning are shown in FIG. 2.

It may be seen from FIG. 2 that a correlation coefficient between the predicted value of the model and the measured value is 0.80, it is indicated that the established support vector machine regression model is relatively high in the accuracy of the internal cross-validation results, and it is further indicated that the established model is more credible.

The above are only preferred embodiments of the present invention, and do not have any limiting effects on the present invention. Any persons skilled in the art, within a scope without departing from the technical schemes of the present invention, make any forms of equivalent replacements or modifications and other changes to the technical schemes and technical content disclosed in the present invention, these changes do not depart from the content of the technical schemes of the present invention, and still fall within a scope of protection of the present invention.

**Claims**

1. A quantitative prediction method for a sensory evaluation score of a delicious degree and a liquid generating sense of a solid-state compound seasoning, **characterized in that**, the contents of 7 components IMP, GMP, $Na^+$, Asp, Glu, Gly and Ala in a plurality of solid-state compound seasonings are used as independent variables; each solid-state compound seasoning is artificially scored for the delicious degree and the liquid generating sense, and a formula is used to comprehensively process the contents of 7 independent variables; comprehensively processed data and the liquid generating sense are quantitatively modeled, to generate a quantitative prediction model for sensory evaluation of the delicious degree and the liquid generating sense of the solid-state compound seasoning; and

after the contents of 7 independent variables in the solid-state compound seasoning to be detected are comprehensively processed, it is substituted into the quantitative prediction model, to obtain the sensory evaluation score of the delicious degree and the liquid generating sense of the solid-state compound seasoning to be detected.

2. The quantitative prediction method for the sensory evaluation score of the delicious degree and the liquid generating sense of the solid-state compound seasoning according to claim 1, **characterized in that**, the comprehensive processing comprises using the following conversion equation to process the parameter content:

$$X_1=+0.260[IMP]+4.955E\text{-}2[GMP]+1.971E\text{-}2[Na]+0.271[Asp]-2.521E\text{-}3[Glu]+0.453[Gly]+1.169[Ala]-0.515;$$

$$X_2=+0.795[IMP]+0.340[GMP]+0.133[Na]-0.617[Asp]-4.217E\text{-}3[Glu]-0.765[Gly]+0.806[Ala]-1.488;$$

$$X_3=+2.354[IMP]+1.660[GMP]-0.103[Na]-0.340[Asp]+2.758E\text{-}3[Glu]-0.663[Gly]+0.686[Ala]-0.862;$$

$$X_4=-0.257[IMP]-1.850[GMP]-0.179[Na]-3.978E\text{-}2[Asp]+3.134E\text{-}3[Glu]-1.035[Gly]+0.665[Ala]+3.013;$$

$$X_5=+2.457[IMP]-2.734[GMP]+0.130[Na]+0.169[Asp]+2.082E\text{-}3[Glu]-0.106[Gly]+6.545E\text{-}2[Ala]-3.024;$$

$$X_6=+5.119[IMP]-5.251[GMP]-6.594E\text{-}2[Na]-5.659E\text{-}2[Asp]-9.137E\text{-}4[Glu]+0.132[Gly]-0.122[Ala]+1.209;$$

$$X_7=+0.917[IMP]+0.463[GMP]-2.781E\text{-}2[Na]+1.008[Asp]-3.006E\text{-}3[Glu]-0.719[Gly]-0.216[Ala]+0.754;$$

in the formula, $X_1$, $X_2$, $X_3$, $X_4$, $X_5$, $X_6$ and $X_7$ respectively represent 7 modeling data after the comprehensive processing of each solid-state compound seasoning.

3. The quantitative prediction method for the sensory evaluation score of the delicious degree and the liquid generating sense of the solid-state compound seasoning according to claim 1, **characterized in that**, before the modeling, the number of the solid-state compound seasonings is greater than or equal to 70, and a taster tastes each solid-state compound seasoning and scores for the delicious degree and the liquid generating sense.

4. The quantitative prediction method for the sensory evaluation score of the delicious degree and the liquid generating sense of the solid-state compound seasoning according to claim 1, **characterized in that**, in each solid-state compound seasoning, 7 independent variable data after the comprehensive processing and the delicious degree and the liquid generating sense are quantitatively modeled using a support vector machine regression algorithm.

5. The quantitative prediction method for the sensory evaluation score of the delicious degree and the liquid generating sense of the solid-state compound seasoning according to claim 4, **characterized in that**, in the support vector machine regression algorithm, a radial basis kernel function is selected, a penalty factor is selected as 13, and an insensitive function is selected as 0.06.

FIG. 1

FIG. 2

**EP 4 134 963 A1**

Europäisches
Patentamt
European
Patent Office
Office européen
des brevets

**EUROPEAN SEARCH REPORT**

Application Number

EP 22 18 6188

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,P | CN 113 609 775 A (SHANGHAI TOTOLE FOOD LTD; UNIV SHANGHAI OCEAN) 5 November 2021 (2021-11-05) * the whole document * ----- | 1-5 | INV. G16C20/30 ADD. G16C20/70 G06N20/10 |
| A | WANG WENLI ET AL: "Characterization and evaluation of umami taste: A review", TRAC TRENDS IN ANALYTICAL CHEMISTRY, ELSEVIER, AMSTERDAM, NL, vol. 127, 30 March 2020 (2020-03-30), XP086190249, ISSN: 0165-9936, DOI: 10.1016/J.TRAC.2020.115876 [retrieved on 2020-03-30] * Section 2.1 The main umami (enhancing) substances and their food source * * Section 4 Evaluation of umami intensity * ----- | 1-5 | |
| A | LIU YA ET AL: "Calculated Taste Activity Values and Umami Equivalences Explain Why Dried Sha-chong ( Sipunculus nudus ) Is a Valuable Condiment", JOURNAL OF AQUATIC FOOD PRODUCT TECHNOLOGY, vol. 25, no. 2, 15 January 2016 (2016-01-15), pages 177-184, XP093009335, US ISSN: 1049-8850, DOI: 10.1080/10498850.2013.839591 * the whole document * ----- | 1-5 | TECHNICAL FIELDS SEARCHED (IPC) G16C G06N |
| A | CN 111 323 543 A (SHANGHAI TOTOLE FOOD LTD) 23 June 2020 (2020-06-23) * the whole document * ----- -/-- | 1-5 | |

The present search report has been drawn up for all claims

| Place of search | Date of completion of the search | Examiner |
|---|---|---|
| The Hague | 19 December 2022 | Denoual, Matthieu |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 1 of 2

11

Europäisches
Patentamt

European
Patent Office

Office européen
des brevets

# EUROPEAN SEARCH REPORT

Application Number

EP 22 18 6188

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | KR 102 017 169 B1 (NAT UNIV CHUNGBUK IND ACAD COOP FOUND [KR] ET AL.) 3 September 2019 (2019-09-03) * the whole document * | 1-5 | |

TECHNICAL FIELDS
SEARCHED (IPC)

The present search report has been drawn up for all claims

| Place of search | Date of completion of the search | Examiner |
|---|---|---|
| The Hague | 19 December 2022 | Denoual, Matthieu |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

page 2 of 2

## ANNEX TO THE EUROPEAN SEARCH REPORT
## ON EUROPEAN PATENT APPLICATION NO.

EP 22 18 6188

This annex lists the patent family members relating to the patent documents cited in the above-mentioned European search report.
The members are as contained in the European Patent Office EDP file on
The European Patent Office is in no way liable for these particulars which are merely given for the purpose of information.

19-12-2022

| Patent document cited in search report | | Publication date | Patent family member(s) | Publication date |
|---|---|---|---|---|
| CN 113609775 | A | 05-11-2021 | NONE | |
| CN 111323543 | A | 23-06-2020 | NONE | |
| KR 102017169 | B1 | 03-09-2019 | NONE | |

EPO FORM P0459

For more details about this annex : see Official Journal of the European Patent Office, No. 12/82